# EUROPEAN PATENT SPECIFICATION

(11) **EP 2 133 076 B1**
(45) Date of publication and mention of the grant of the patent: **21.03.2012**
(21) Application number: 08425422.6
(22) Date of filing: 12.06.2008
(51) Int. Cl.: A61K 31/343

(54) **Compositions for the treatment and prevention of infections of the oral cavity**
Zusammensetzungen zur Behandlung und Vorbeugung von Infektionen der Mundhöhle
Compositions pour la prévention et le traitement des infections de la cavité buccale

(43) Date of publication of application: 16.12.2009
(73) Proprietor: INDENA S.p.A., 20139 Milano (IT)
(72) Inventor: Bombardelli, Ezio, 27027 Gropello Cairoli (PV) (IT); Fontana, Gabriele, 20139 Milano (IT); Giori, Andrea, 20139 Milano (IT); Morazzoni, Paolo, 20139 Milano (IT); Riva, Antonella, 20139 Milano (IT); Ronchi, Massimo, 20139 Milano (IT)
(74) Representative: Minoja, Fabrizio

(56) References cited:
- EP-A- 0 297 535
- EP-A- 0 464 297
- US-A- 4 908 211
- US-A- 5 066 483
- DATABASE WPI Week 198850 Thomson Scientific, London, GB; AN 1988-357157 XP002500466 & JP 63 267714 A (SATO SEIYAKU KK) 4 November 1988 (1988-11-04)

## Description

### SUMMARY

The present invention relates to compositions based on benzophenanthridine alkaloids, benzofuran compounds and catechin polyphenols, which are useful in the treatment and prevention of infections of the oral cavity.

The compositions of the present invention possess antibacterial, antifungal and antienzymatic activities, which are useful in oral hygiene and the treatment and prevention of pathological forms of various origins associated with the dentition, implants and complications of surgery of the oral cavity.

Said compositions can be administered in the form of tablets that dissolve slowly in the oral cavity, or in the form of a mouthwash or chewing gum.

### Prior art

The formation of bacterial or fungal films in the mouth very often gives rise to disorders such as tooth decay, pyorrhoea and periodontal and gingival infections. In particular, the formation of bacterial or fungal films is very common in implantology, denture maintenance, and elderly dental patients in general.

It has been reported in the literature that benzophenanthridine alkaloids possess antibacterial and antifungal activity, in particular against Gram+ bacteria, and are able to rupture the bacterial film, thus making the pathogen sensitive to bacteriostatic compounds or antibiotics.

Benzofuran compounds with a neolignan structure are also known to inhibit the formation of bacterial and fungal films or rupture films already formed, thus preventing their re-formation; such compounds are found in extracts of *Krameria triandra, Eupomatia laurina* and *Piper sp*, in particular eupomatenoids and 2-(2',4'-dihydroxyphenyl)-5-propenylbenzofuran.

Enzymes such as α-amylase, glucosidase and takadiastase which are present in the oral cavity promote the breakdown of carbohydrates; however, this leads to the formation of glucose which is very harmful to oral hygiene because it contributes to the formation of bacterial film in the oral cavity. Inhibitors of these enzymes therefore help to prevent the formation of the bacterial film.

Procyanidins, in particular catechin oligomers esterified with gallic acid, are glucosidase and takadiastase inhibitors.

The polyphenols extracted from *Vitis vinifera* are powerful inhibitors of α-amylase and glucosidase.

### DESCRIPTION OF THE INVENTION

The present invention relates to compositions based on:
a) benzophenanthridine alkaloids;
b) benzofuran compounds; and
c) catechin polyphenols;
with antibacterial, antifungal and antienzymatic activities, which reduce the formation of bacterial and fungal films in the oral cavity, thus reducing halitosis and the formation of dental plaque.

The preferred benzophenanthridine alkaloids are chelerythrine and sanguinarine, while the preferred benzofuran compounds are compounds with a neolignan structure, found in extracts of *Krameria triandra*, *Eupomatia laurina* and *Piper sp*,in particular eupomatenoids and 2-(2',4'-dihydroxyphenyl)-5-propenylbenzofuran.

According to the invention, the benzofuran compounds have the following formula 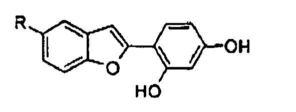 where R may be hydrogen or a linear or branched alkyl chain with 2 to 6 carbon atoms, or an alkyl chain substituted by amino, nitro groups; R is preferably hydrogen or C1-C3 alkyl.

Said benzofuran compounds are known and can be prepared by conventional methods, for example by reaction of a phenol suitably substituted with 2-phenoxy-2',4'-dimethoxyacetophenone in the conditions reported in Chimie Therapeutique 1973, 8, 398, followed by cyclisation in the presence of polyphosphoric acid in xylene and hydrolysis of the methoxy and hydroxy groups.

It has now surprisingly been found that the compositions according to the invention possess an extremely powerful antibacterial, antifungal and antienzymatic activity, greater than that obtained from the sum of the various components administered separately. Said effect may be due to a synergistic action mechanism which takes place between the various components of the association in question.

The compositions according to the invention are therefore useful in oral hygiene and in the treatment and prevention of pathological forms of various origins associated with the dentition, implantology and complications of surgery of the oral cavity.

More particularly, the present invention relates to compositions based on:
a) benzophenanthridine alkaloids selected from sanguinarine and/or chelerythrine and/or derivatives thereof;
b) benzofuran compounds as defined above;
c) monomeric or oligomeric catechin polyphenols.

According to the invention, the compositions will contain the various components in the following intervals (by weight per unit dose):
a) benzophenanthridine alkaloids: from 0.5 mg to 10 mg;
b) benzofuran: from 5 to 25 mg;
c) polyphenol compounds: from 10 to 100 mg.

According to a particularly preferred aspect, the compositions will contain the various components within the following intervals (by weight per unit dose):
a) benzophenanthridine alkaloids: from 2.5 to 5 mg;
b) benzofuran compounds: from 3 to 10 mg;
c) polyphenol compounds: from 40 to 50 mg.

The benzophenanthridine alkaloids sanguinarine and chelerythrine may be present in the free or salified form, as such in substantially pure form or in the form of extracts of *Sanguinaria canadensis, Macleaya cordata* or *Macleaya macrocarpa*. According to a preferred aspect, the benzophenanthridine alkaloids will be present in a form salified with luteic acid. Said salts, which are prepared by reacting the sulphates or chlorides of the alkaloids with the sodium or potassium salt of luteic acid and subsequent crystallisation, have proved particularly effective for the purposes of the present invention.

The compounds with a benzofuran structure described above may be present as such or in the form of extracts containing them, such as extracts of *Krameria triandra, Eupomatia laurina* and *Piper sp.* The compounds isolated from *Krameria triandra* which have proved particularly active are Eupomatenoid 6 and neolignan 2-(2,4-dihydroxyphenyl)-5-(E)-propenylbenzofuran, which have demonstrated antibacterial and antifungal activity on numerous strains of Gram+ bacteria, fungi and anaerobic bacteria.

The polyphenol compounds may be present in the form of monomer units such as catechin, epicatechin and gallocatechin and its gallic esters at the C3 hydroxyl, or oligomeric units, preferably up to five units. According to a particularly preferred aspect, the oligomer units will be esterified with gallic acid in C3. The polyphenol compounds may also be present in the form of extracts of seeds or aerial parts of *Vitis vinifera, Aesculus hippocastanum, Camellia sinensis, Theobroma cacao* and the like. These compounds are particularly indicated in oral hygiene, halitosis and gingival infections.

The compositions according to the invention will be conveniently formulated as melt-in-the-mouth tablets, mouthwashes, gels for dispersal in the oral cavity, chewing gums and the like. Said formulations can be prepared according to well-known conventional methods, such as those described in "Remington's Pharmaceutical Handbook", Mack Publishing Co., N.Y., USA, together with suitable excipients.

The formulations according to the invention will be administered to patients up to 5 times in 24 hours for several days, depending on the disorder to be treated. For oral hygiene alone, the number of administrations could be reduced to 2, taken at main meals.

The examples set out below illustrate the invention, without limiting its scope.

### Example 1 - Preparation of benzofuran compounds

### Step A. Preparation of 2-phenoxy-2',4'-dimethoxyacetophenone (a)

A solution of 2-bromo-2',4'-dimethoxyacetophenone (5 g, 19.1 mmols) in 25 mL of 2-butanone was added to a suspension of phenol (1.8 g, 19.1 mmols), K₂CO₃ (2.6 g, 19.1 mmols) and Kl (41.5 mg, 0.25 mmols) in 20.0 mL of the same solvent. The solution was then refluxed for 20 hours. The mixture was filtered and the solvent was evaporated off under vacuum. The residue obtained was dissolved in EtOAc and washed with a 10% aqueous solution of NaOH and then with water. The organic extract was dried over Na₂SO_{4,} filtered and evaporated under vacuum. Finally, the crude residue was washed with Et₂O and dried under low pressure to provide 4.4 g (yield: 84%) of the title compound.

### Step B. Preparation of 2-(2',4'-dimethoxyphenyl)benzofuran (b)

12 g of polyphosphoric acid was added to a solution of the compound obtained at Step A (4.4 g, 16.2 mmols) in 130.0 mL of xylene. The mixture was refluxed for 2 hours, and then left to cool at room temperature. The solution was then decanted and evaporated under low pressure. The resulting residue (3.7 g, yield: 90%) was used in the next step without further purification.

### Step C. Preparation of-(2',4'-dihydroxyphenyl)benzofuran (1)

A mixture of the compound prepared at Step B (3.7 g, 14.5 mmols) and pyridine hydrochloride (11.1 g, 96.4 mmols) was heated to 225°C for 45 minutes. The red product formed was poured into 10% HCl. The mixture was washed repeatedly with EtOAc; the combined organic layers were dried over Na₂SO₄ and evaporated. The residue was purified by column chromatography (hexane/EtOAc= 7:3) to provide. The final compound was obtained in a 41 % yield (1.36 g) after crystallisation from benzene.

| **Formulation example 1 - Melt-in-the-mouth tablets** | |
|---|---|
| Benzophenanthridine alkaloid | 2.5 mg |
| Benzofuran compound | 10.0 mg |
| Extract of *Vitis vinifera* | 50.0 mg |
| Xylitol | 500.0 mg |
| Mannitol | 400.0 mg |
| Liquorice flavouring | 50.0 mg |
| Magnesium stearate | 10.0 mg |
| Acesulfame K | 5.0 mg |

| **Formulation example 2 - Oral gel** | |
|---|---|
| Benzophenanthridine alkaloid | 3.0 mg |
| Benzofuran compound | 10.0 mg |
| Extract of *Camellia sinensis* | 50.0 mg |
| Glycerin | 400.0 mg |
| Liquid sorbitol | 200.0 mg |
| Hydroxyethylcellulose | 30.0 mg |
| Mint flavouring | 20.0 mg |
| Methyl para-hydroxy benzoate | 10.0 mg |
| Acesulfame K | 5.0 mg |
| Purified water q.s. to | 2.0 g |

## Claims

1. Compositions based on:
a) benzophenanthridine alkaloids
b) benzofuran compounds of formula 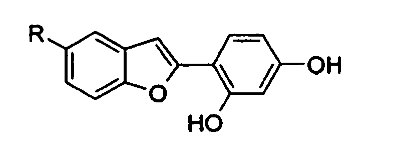 where R may be hydrogen or a linear or branched alkyl chain with 2 to 6 carbon atoms, or an alkyl chain substituted by amino, nitro groups;
c) catechin polyphenols.

2. Compositions as claimed in claim 1, based on:
a) benzophenanthridine alkaloids selected from sanguinarine and/or chelerythrine and/or derivatives thereof;
b) benzofuran compounds as specified above, or extracts containing them;
c) monomeric or oligomeric catechin polyphenols.

3. Compositions as claimed in claims 1 and 2, wherein the various components are present in the following intervals (by weight per unit dose):
a) benzophenanthridine alkaloids: from 0.5 mg to 10 mg;
b) benzofuran compounds: from 5 to 25 mg;
c) polyphenol compounds: from 10 to 100 mg.

4. Compositions as claimed in claim 3, wherein the various components are present in the following intervals (by weight per unit dose):
a) benzophenanthridine alkaloids: from 2.5 to 5 mg;
b) benzofuran compounds: from 3 to 10 mg;
c) polyphenol compounds: from 40 to 50 mg.

5. Compositions as claimed in the preceding claims, wherein the benzophenanthridine alkaloids sanguinarine and chelerythrine are present in the free or salified form, as such in substantially pure form or in the form of extracts of *Sanguinaria canadensis, Macleaya cordata* or *Macleaya macrocarpa*.

6. Compositions as claimed in claim 5, wherein the benzophenanthridine alkaloids are present in the salified form with luteic acid.

7. Compositions as claimed in claims '1-4, wherein the benzofuran compounds are present as such or in the form of extracts containing them.

8. Compositions as claimed in claim 7, wherein the benzofuran compounds are present in the form of extracts of *Krameria triandra*, *Eupomatia laurina* and *Piper sp.*

9. Compositions as claimed in claims 1-4, wherein the polyphenol compounds are present in the form of monomer units such as catechin, epicatechin and gallocatechin and its gallic esters at the C3 hydroxyl, or oligomer units.

10. Compositions as claimed in claim 9, wherein the polyphenol compounds are present in the form of extracts of seeds or aerial parts of *Vitis vinifera, Aesculus hippocastanum, Camellia sinensis* or *Theobroma cacao.*

11. Formulations as claimed in any one of claims 1 to 10, in the form of melt-in-the-mouth tablets, mouthwashes, gels to be dispersed in the oral cavity, chewing gums and the like.

12. The use of:
a) benzophenanthridine alkaloids;
b) benzofuran compounds;
and
c) catechin polyphenols;
for the preparation of oral formulations for the oral hygiene and for the treatment and prevention of disorders of the oral cavity of various origins.

13. The use as claimed in claim 12, wherein the disorders of the oral cavity are pathological forms associated with the dentition, implants or complications of surgery of the oral cavity.

## Patentansprüche

1. Zusammensetzungen basierend auf:
a) Benzophenanthridin-Alkaloiden
b) Benzofuranverbindungen der Formel 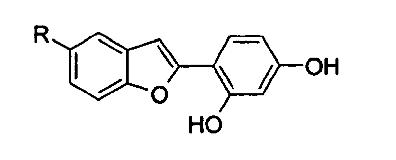 worin R Wasserstoff oder eine lineare oder verzweigte Alkylkette mit 2 bis 6 Kohlenstoffatomen, oder eine Alkylkette, die durch Amino-, Nitro-Gruppen substituiert ist, sein kann;
c) Catechinpolyphenole.

2. Zusammensetzungen gemäß Anspruch 1, basierend auf:
a) Benzophenantridin-Alkaloiden ausgewählt aus Sanguinarin und/oder Chelerythrin und/oder Derivaten davon;
b) Benzofuranverbindungen, wie oben angegeben, oder sie enthaltenden Extrakten;
c) monomeren oder oligomeren Catechinpolyphenolen.

3. Zusammensetzungen gemäß den Ansprüchen 1 und 2, wobei die verschiedenen Komponenten in den folgenden Mengenbereichen (Gewicht pro Einheitsdosis) vorliegen:
a) Benzophenanthridin-Alkaloide: von 0,5 mg bis 10 mg;
b) Benzofuranverbindungen: von 5 bis 25 mg;
c) Polyphenolverbindungen: von 10 bis 100 mg.

4. Zusammensetzungen gemäß Anspruch 3, wobei die verschiedenen Komponenten in den folgenden Mengenbereichen (Gewicht pro Einheitsdosis) vorliegen:
a) Benzophenanthridin-Alkaloide: von 2,5 bis 10 mg;
b) Benzofuranverbindungen: von 3 bis 10 mg;
c) Polyphenolverbindungen: von 40 bis 50 mg.

5. Zusammensetzungen gemäß den vorstehenden Ansprüchen, wobei die Benzophenanthridin-Alkaloide Sanguinarin und Chelerythrin in freier Form oder als Salz, als solche in im wesentlichen reiner Form oder in Form von Extrakten aus *Sanguinaria canadensis, Macleaya cordata* oder *Macleaya macrocarpa,* vorliegen.

6. Zusammensetzungen gemäß Anspruch 5, wobei die Benzophenanthridin-Alkaloide in Salzform mit Luteinsäure vorliegen.

7. Zusammensetzungen gemäß den Ansprüchen 1 bis 4, wobei die Benzofuranverbindungen als solche oder in Form von Extrakten, welche sie enthalten, vorliegen.

8. Zusammensetzungen gemäß Anspruch 7, wobei die Benzofuranverbindungen in Form von Extrakten aus *Krameria triandra, Eupomatia laurina* und *Piper sp.* vorliegen.

9. Zusammensetzungen gemäß den Ansprüchen 1 bis 4, wobei die Polyphenolverbindungen in Form von Monomereinheiten wie Catechin, Epicatechin und Gallocatechin und deren Gallussäure-Estern am C3-Hydroxyl, oder von Oligomereinheiten, vorliegen.

10. Zusammensetzungen gemäß Anspruch 9, wobei die Polyphenolverbindungen in Form von Extrakten aus Samen oder oberirdischen Teilen von *Vitis vinifera, Aesculus hippocastanum, Camellia sinensis* oder *Theobroma cacao* vorliegen.

11. Formulierungen gemäß einem der Ansprüche 1 bis 10, in Form von im Mund zergehenden Tabletten, Mundwässern, in der Mundhöhle zu dispergierenden Gelen, Kaugummis und dergleichen.

12. Verwendung von:
a) Benzophenantridin-Alkaloiden;
b) Benzofuranverbindungen;
und
c) Catechinpolyphenolen;
zur Herstellung von oralen Formulierungen für die orale Hygiene und für die Behandlung und Verhütung von Störungen der Mundhöhle unterschiedlicher Herkunft.

13. Verwendung gemäß Anspruch 12, wobei die Störungen der Mundhöhle pathologische Formen, in Zusammenhang mit Zahnung, Implantaten oder Komplikationen bei chirurgischen Eingriffen der Mundhöhle, sind.

## Revendications

1. Compositions basées sur :
a) des alcaloïdes de benzophénanthridine
b) des composés de benzofuranne de formule 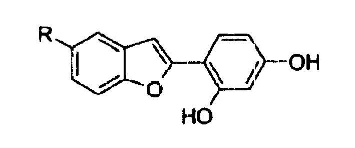 où R peut être un atome d'hydrogène ou une chaîne d'alkyle linéaire ou ramifiée avec 2 à 6 atomes de carbone, ou une chaîne d'alkyle substituée par des groupes amino, nitro ;
c) des polyphénols catéchiques.

2. Compositions selon la revendication 1, basées sur :
a) des alcaloïdes de benzophénanthridine choisis parmi de la sanguinarine et/ou de la chélérythrine et/ou leurs dérivés ;
b) des composés de benzofuranne tels que spécifiés ci-dessus, ou des extraits les contenant ;
c) des polyphénols catéchiques monomères ou oligomères.

3. Compositions selon les revendications 1 et 2, dans lesquelles les divers composants sont présents dans les intervalles suivants (en poids par dose unitaire) :
a) alcaloïdes de benzophénanthridine : de 0,5 mg à 10 mg ;
b) composés de benzofuranne : de 5 à 25 mg ;
c) composés de polyphénol : de 10 à 100 mg.

4. Compositions selon la revendication 3, dans lesquelles les divers composants sont présents dans les intervalles suivants (en poids par dose unitaire) :
a) alcaloïdes de benzophénanthridine : de 2,5 à 5 mg ;
b) composés de benzofuranne : de 3 à 10 mg ;
c) composés de polyphénol : de 40 à 50 mg.

5. Compositions selon les revendications précédentes, dans lesquelles les alcaloïdes de benzophénanthridine, la sanguinarine et la chélérythrine, sont présents dans la forme libre ou salifiée, telle que dans une forme essentiellement pure ou bien sous la forme d'extraits de *Sanguinaria canadensis,* de *Macleaya cordata* ou de *Macleaya macrocarpa.*

6. Compositions selon la revendication 5, dans lesquelles les alcaloïdes de benzophénanthridine sont présents dans la forme salifiée avec de l'acide lutéique.

7. Compositions selon les revendications 1 à 4, dans lesquelles les composés de benzofuranne sont présents tels que ou bien sous la forme d'extraits les contenant.

8. Compositions selon la revendication 7, dans lesquelles les composés de benzofuranne sont présents sous la forme d'extraits de *Krameria triandra, d'Eupomatia laurina* et de *Piper sp.*

9. Compositions selon les revendications 1 à 4, dans lesquelles les composés de polyphénol sont présents sous la forme d'unités monomères tels que la catéchine, l'épicatéchine et la gallocatéchine et ses esters galliques au niveau de l'hydroxyle sur la position C3, ou d'unités oligomères.

10. Compositions selon la revendication 9, dans lesquelles les composés de polyphénol sont présents sous la forme d'extraits de graines ou de parties aériennes de *Vitis vinifera*, d'*Aesculus hippocastanum,* de *Camellia sinensis* ou de *Theobroma cacao.*

11. Formulations selon l'une quelconque des revendications 1 à 10, sous la forme de comprimés à laisser fondre dans la cavité buccale, de collutoires, de gels à disperser dans la cavité orale, de gommes à mâcher et analogues.

12. Utilisation de :
a) alcaloïdes de benzophénanthridine ;
b) composés de benzofuranne ;
et
c) polyphénols catéchiques ;
pour la préparation de formulations orales pour l'hygiène orale et pour le traitement et la prévention de troubles de la cavité orale d'origines diverses.

13. Utilisation selon la revendication 12, dans laquelle les troubles de la cavité orale sont des formes pathologiques associées avec la denture, les implants ou les complications d'une chirurgie de la cavité orale.
